# EUROPEAN PATENT APPLICATION

(11) **EP 4 400 028 A1**
(43) Date of publication of application: **17.07.2024**
(21) Application number: 24151186.4
(22) Date of filing: 10.01.2024
(51) Int. Cl.: A61B 1/00, A61B 1/12

(54) **MEDICAL INSTRUMENT GUIDE DEVICE**

(30) Priority: 10.01.2023 JP 2023001429
(71) Applicant: FUJIFILM Corporation, Tokyo 106-8620 (JP)
(72) Inventor: ABE, Shinya, Tokyo, 106-8620 (JP); FURUTA, Ayana, Tokyo, 106-8620 (JP); KAWASHIMA, Ayumu, Tokyo, 106-8620 (JP)
(74) Representative: Hughes, Andrea Michelle

(57) **Abstract**

Provided is a medical instrument guide device that can improve the operability of a medical instrument.

A guide tube of the embodiment includes a long tube main body (50) to be inserted into a body, and a handle part (100) provided on a proximal end side of the tube main body, in which the handle part includes an inner sheath (110) to which a proximal end part (110B) of the tube main body is connected and a protection sheath (120) externally inserted into the inner sheath, and the inner sheath includes a port part (112) for supplying and discharging a fluid to and from the tube main body.

## Description

### BACKGROUND OF THE INVENTION

### 1. Field of the Invention

The present invention relates to a medical instrument guide device for guiding a medical instrument, such as an endoscope insertion part, into a body.

### 2. Description of the Related Art

An insertion part of an endoscope (hereinafter, also referred to as an "endoscope insertion part") is inserted along a winding and flexible insertion path such as an upper digestive tract or a lower digestive tract. Therefore, the endoscope insertion part has the flexibility that can be inserted along the insertion path.

However, since the intestinal tract, which is an example of the insertion path, has a portion that is not fixed to the body (for example, a sigmoid colon) and the above-described portion is deformed in the middle even though the insertion part is pushed during insertion of the endoscope insertion part, there is a problem that it is difficult for a distal end part of the insertion part to move forward. Further, there is a case where a treatment tool is led out from the distal end part of the insertion part to perform a precise operation of cutting out a lesion portion during treatment of the endoscope, but there is a case where the deformation of the above-described portion interferes with the precise operation.

Therefore, JP2021-531111A discloses a hardening device system that can eliminate an unstable operation during insertion and during treatment. The hardening device system in JP2021-531111A includes a hardening device having walls comprising a plurality of layers including a braided layer, an outer layer, and an inner layer, and further includes a handle having a vacuum or pressure inlet that supplies vacuum or pressure to the hardening device. In addition, an operation element of the hardening device system is used to turn on and off vacuum or pressure to transition the hardening device between flexible and hardening configurations.

### SUMMARY OF THE INVENTION

However, since the handle (handle part) of the hardening device system (medical instrument guide device) in JP2021-531111A has a complicated shape, it is difficult for an operator to grip the handle part, and as a result, there is a problem that the operability of the medical instrument is affected.

The present invention has been made in view of such circumstances, and is to provide a medical instrument guide device that can improve the operability of a medical instrument.

In order to solve the above problems, an aspect of the present invention relates to a medical instrument guide device having an insertion passage for guiding a medical instrument into a body, the medical instrument guide device comprising: a long tube main body to be inserted into the body; and a handle part provided on a proximal end side of the tube main body, in which the handle part includes an inner sheath to which a proximal end part of the tube main body is connected and a protection sheath externally inserted into the inner sheath, and the inner sheath includes a port part for supplying and discharging a fluid to and from the tube main body.

According to an embodiment, it is preferable that the tube main body includes a hardness change part that is arranged along a longitudinal direction of the tube main body and of which hardness is changed due to the fluid supplied and discharged from the port part.

According to an embodiment, it is preferable that a stress relaxation ring that surrounds an outer periphery of a connection portion between the inner sheath and the tube main body in a loosely fitted state is arranged, and the inner sheath and the protection sheath are coupled and fixed to the stress relaxation ring.

According to an embodiment, it is preferable that a leakage prevention valve that prevents a liquid from leaking from the tube main body is arranged inside the handle part.

According to an embodiment, it is preferable that the protection sheath includes a flange-shaped valve pressing part provided on an inner peripheral surface of the protection sheath, and the leakage prevention valve is interposed between the valve pressing part and a proximal end part of the inner sheath so that a position of the handle part in an axial direction is restricted.

According to an embodiment, it is preferable that an engaging part is provided to protrude on an outer peripheral surface of the inner sheath, the protection sheath includes a slit-shaped engaged part open toward a tube main body side, and the protection sheath is externally inserted into the inner sheath in a state in which rotation of the protection sheath is locked with respect to the inner sheath by engaging the engaged part and the engaging part with each other.

According to an embodiment, it is preferable that the port part is provided in the engaging part.

According to an embodiment, it is preferable that the tube main body includes a hardness change part that is arranged along a longitudinal direction of the tube main body and of which hardness is changed due to the fluid supplied and discharged from the port part, and the port part includes a fluid port for supplying and discharging the fluid to and from the tube main body, and a liquid port for supplying a liquid to an inner peripheral surface of the insertion passage.

According to an embodiment, it is preferable that the fluid port and the liquid port are installed adjacent to each other along an axial direction of the inner sheath.

According to an embodiment, it is preferable that the liquid port is arranged on a side opposite to a side of the inner sheath to which the tube main body is connected, with respect to the fluid port.

According to an embodiment, it is preferable that the medical instrument guide device further comprise: a fluid tube connected to the fluid port; and a liquid tube connected to the liquid port, in which a pull-out direction of a proximal end side portion of the fluid tube from the fluid port and a pull-out direction of a proximal end side portion of the liquid tube from the liquid port are the same direction as each other.

According to an embodiment, it is preferable that the pull-out direction of the proximal end side portion of the fluid tube from the fluid port and the pull-out direction of the proximal end side portion of the liquid tube from the liquid port are directions perpendicular to the axial direction of the inner sheath.

According to an embodiment, it is preferable that the protection sheath includes a first sleeve part arranged on a tube main body side, and a second sleeve part arranged on a side opposite to the tube main body side with respect to the first sleeve part and having a larger outer diameter than the first sleeve part.

According to an embodiment, it is preferable that the first sleeve part includes a sleeve body large-diameter part arranged on the tube main body side, and a sleeve body small-diameter part arranged on a side opposite to the tube main body side with respect to the sleeve body large-diameter part and having a smaller outer diameter than the sleeve body large-diameter part.

According to an embodiment, it is preferable that an axial length of the sleeve body small-diameter part is longer than an axial length of the second sleeve part.

According to an embodiment, it is preferable that the sleeve body small-diameter part is arranged on a side opposite to the tube main body side with respect to the port part.

According to an embodiment, it is preferable that the tube main body includes a hardness change part that is arranged along a longitudinal direction of the tube main body and of which hardness is changed due to the fluid supplied and discharged from the port part, the port part includes a fluid port for supplying and discharging the fluid to and from the tube main body, a fluid tube connected to the fluid port is provided, a switching unit is provided in a middle of the fluid tube, and the switching unit includes a switching member capable of switching supply and discharge of the fluid for the fluid port.

According to an embodiment, it is preferable that the switching unit includes a fixing part attachably and detachably fixable to the medical instrument.

According to an embodiment, it is preferable that the medical instrument is an endoscope having an insertion part to be inserted into the body, and the fixing part includes a fitting part capable of being fitted into a treatment tool insertion part of the endoscope.

According to an embodiment, it is preferable that the switching member is arranged on a side opposite to a side of the fluid port with respect to the fixing part.

According to the present invention, the operability of the medical instrument can be improved.

### BRIEF DESCRIPTION OF THE DRAWINGS

Fig. 1 is an external view of a guide tube according to a first embodiment.
Fig. 2 is an enlarged cross-sectional view of a main part showing a configuration of the guide tube.
Fig. 3 is a schematic view showing a configuration of a main part of a spiral tube.
Fig. 4 is an explanatory view showing a state in which the spiral tube is bent.
Fig. 5 is an assembly perspective view of a handle part as viewed from an oblique direction on a proximal end side of an axis.
Fig. 6 is an assembly perspective view of the handle part as viewed from an oblique direction on a distal end side of the axis.
Fig. 7 is a cross-sectional view showing a configuration of the handle part.
Fig. 8 is a perspective view of a three way stopcock provided in the middle of a fluid tube.
Fig. 9 is a perspective view of a main part in which the three way stopcock is attachably and detachably fixed to an endoscope by a hook.
Fig. 10 is an explanatory view showing a treatment example of the endoscope using the guide tube.
Fig. 11 is an explanatory view of a main part showing an operation example of the endoscope using the guide tube according to the first embodiment.
Fig. 12 is an external view showing a main part of a guide tube according to a second embodiment.
Fig. 13 is a perspective view of a protection sheath of Fig. 12 as viewed in an oblique direction on a proximal end side of an axis.
Fig. 14 is a perspective view of the protection sheath of Fig. 12 as viewed in an oblique direction on a distal end side of the axis.
Fig. 15 is an explanatory view of a main part showing an operation example of an endoscope using the guide tube according to the second embodiment.
Fig. 16 is a perspective view showing a modification example for arranging the three way stopcock in the endoscope.

### DESCRIPTION OF THE PREFERRED EMBODIMENTS

Hereinafter, embodiments of a medical instrument guide device of the present invention will be described with reference to the accompanying drawings.

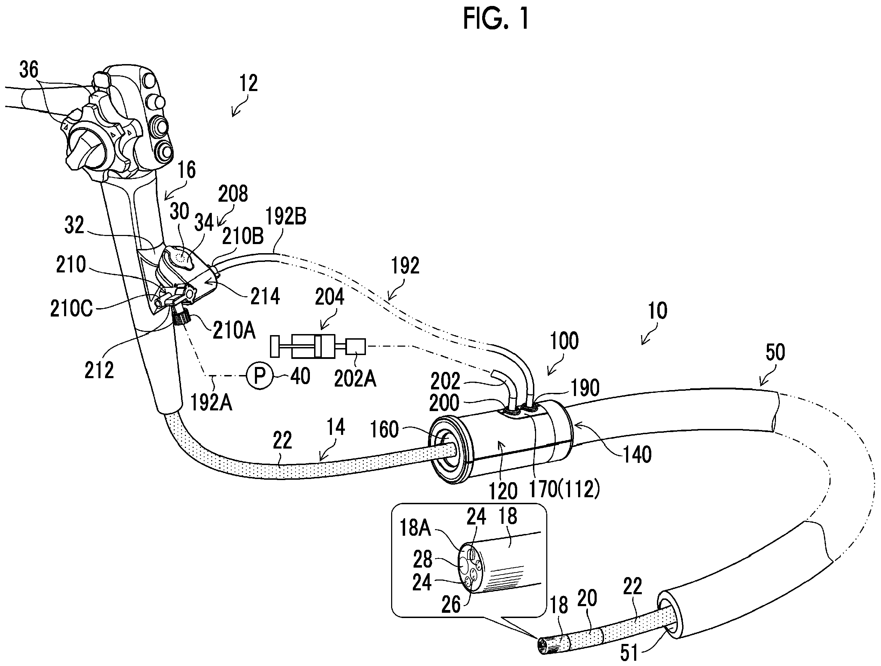

Fig. 1 is an external view of a medical instrument guide device (hereinafter, referred to as a "guide tube") 10 according to a first embodiment of the present invention. Fig. 1 also shows an endoscope 12 in addition to the guide tube 10, and shows a usage form in which an insertion part 14 of the endoscope 12 is inserted into the guide tube 10.

First, the endoscope 12 will be described. The endoscope 12 includes, as shown in Fig. 1, the insertion part 14 and a hand operating part 16, and a proximal end side of the insertion part 14 is coupled to the hand operating part 16. The endoscope 12 is an example of a medical instrument according to the embodiment of the present invention, which is guided into the body by the guide tube 10.

The insertion part 14 is configured by sequentially connecting a distal end hard part 18, a bendable part 20, and a soft part 22 from a distal end side toward the proximal end side. Fig. 1 shows a state in which the distal end sides of the distal end hard part 18, the bendable part 20, and the soft part 22 protrude to the outside from a distal end opening part 51 of a tube main body 50 forming the guide tube 10.

The distal end hard part 18 includes, on a distal end surface 18A of the distal end hard part 18, a pair of illumination windows 24 for illuminating an inside of the body, an observation window 26 for acquiring a video of the inside of the body under the illumination from the illumination windows 24, and a treatment tool outlet port 28 for leading out a treatment tool, such as forceps or a high frequency treatment tool. A treatment tool insertion part 32 is provided to protrude on the hand operating part 16, and the treatment tool described above is inserted toward the treatment tool outlet port 28 from an insertion port 30 provided in the treatment tool insertion part 32. It should be noted that Fig. 1 shows a state in which the insertion port 30 is sealed by a cap 34.

The bendable part 20 is operated to be bent in a desired direction by operating a pair of angle knobs 36 provided in the hand operating part 16. The soft part 22 is formed of a flexible member having the flexibility in a bending direction. Since the configuration of the endoscope 12 is well known in the related art, detailed illustration and the description thereof will be omitted here. Hereinafter, the guide tube 10 according to the first embodiment will be specifically described.

### First embodiment

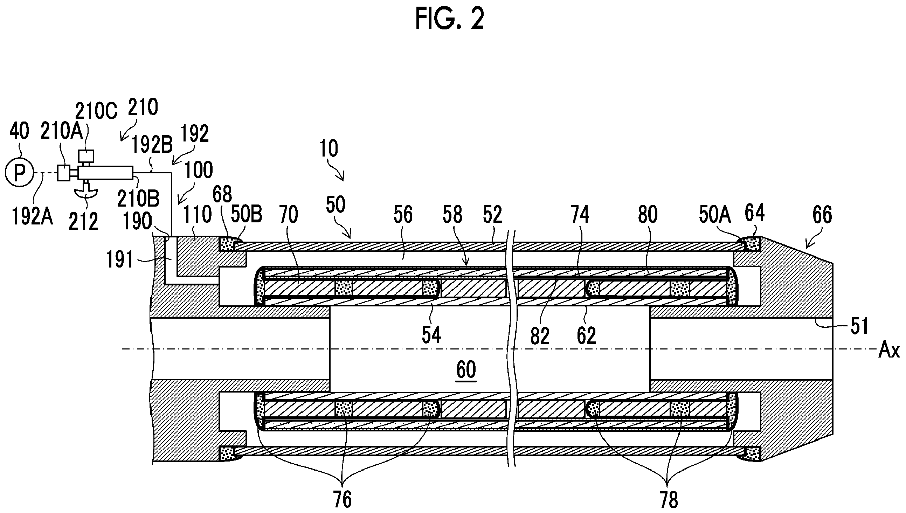

Fig. 2 is an enlarged cross-sectional view of a main part particularly showing a configuration of the tube main body 50 of the guide tube 10 according to the first embodiment. As shown in Fig. 2, the guide tube 10 comprises a long tube main body 50 to be inserted into the body and a handle part 100 (see Fig. 1) gripped by an operator. The respective axes of the tube main body 50 and the handle part 100 are arranged along a direction of an axis Ax of the guide tube 10. Hereinafter, the configuration of the tube main body 50 will be described with reference to Fig. 2. It should be noted that, since Fig. 2 is a schematic view showing the configuration of each member in an easy-to-understand manner, the scale of each member may be different from the actual scale.

As shown in Fig. 2, the tube main body 50 includes a flexible outer tube 52, a flexible inner tube 54 arranged inside the outer tube 52, and a shape deformable body 58 arranged in an inner space 56 between the outer tube 52 and the inner tube 54. Here, an insertion passage 60 of the insertion part 14 (see Fig. 1) is defined by an inner peripheral surface of the inner tube 54. In addition, a hydrophilic coating 62 is formed on the inner peripheral surface of the inner tube 54, and thus a frictional resistance between the inner tube 54 and the insertion part 14 (see Fig. 1) is reduced.

Each of the outer tube 52 and the inner tube 54 is made of, for example, a soft resin material which can be bent along the bending part of the intestinal tract. Examples of the soft resin material include urethane or polyester resin, but the present invention is not limited to this. A thickness of each of the outer tube 52 and the inner tube 54 is, for example, about 100 µm, and a thickness of the inner space 56 positioned between the outer tube 52 and the inner tube 54 is, for example, about 800 µm.

The inner space 56 is a space of which a cross-sectional shape in a direction orthogonal to the axis Ax is formed in an annular shape to surround an outer periphery of the inner tube 54. The inner space 56 is sealed by a tubular cap 66 connected to a distal end part 50A of the tube main body 50 by an adhesive 64 and an inner sheath 110 (described later) connected to a proximal end part 50B of the tube main body 50 by an adhesive 68. It should be noted that the distal end opening part 51 shown in Fig. 1 is provided in the cap 66 of Fig. 2.

The shape deformable body 58 is deformable along the shapes of the outer tube 52 and the inner tube 54, and includes, as an example, a spiral tube 70 arranged along the direction of the axis Ax.

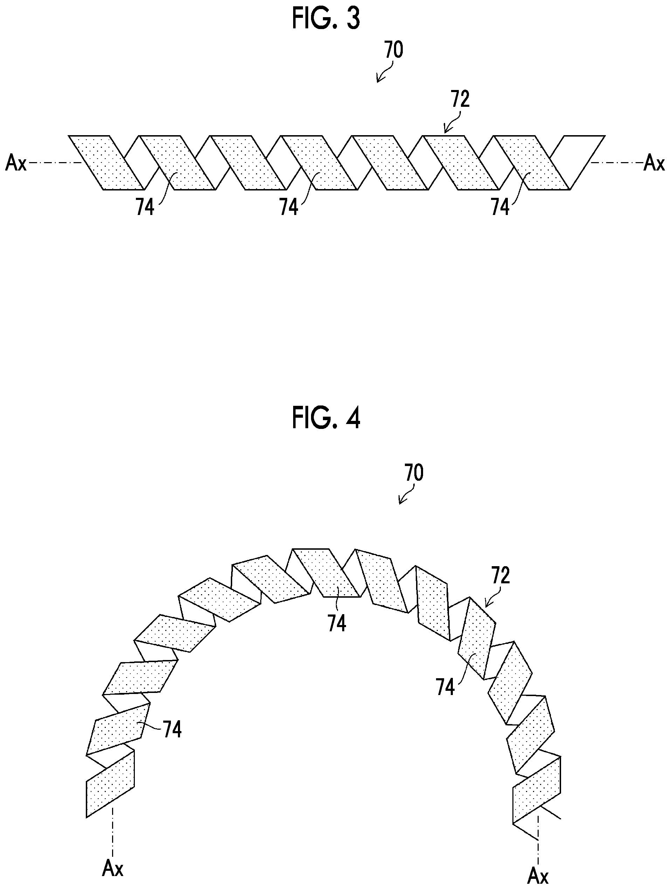

Fig. 3 is a schematic view showing a configuration of a main part of the spiral tube 70. As shown in Fig. 3, the spiral tube 70 is configured by spirally winding a band-shaped member 72 and is provided on an outer peripheral side of the inner tube 54 (see Fig. 2). The band-shaped member 72 is made of steel use stainless (SUS) as an example, but the present invention is not limited to this. In a case where the thickness of the tube main body 50 is, for example, about 1 mm, it is preferable that a thickness of the band-shaped member 72 is 300 µm or less.

A high friction surface 74 is provided on an outer peripheral surface of the spiral tube 70. Examples of the high friction surface 74 include a resin layer obtained by coating an outer peripheral surface of the spiral tube 70 with a resin such as urethane coating or silica coating, a rough surface formed on the outer peripheral surface of the spiral tube 70, and a resin layer obtained by coating the rough surface thereof with a resin. As shown in Fig. 2, in the spiral tube 70 formed as described above, two winding portions on the proximal end side of the spiral tube 70 are adhered to a proximal end side of the inner tube 54 by an adhesive 76, and two winding portions on the distal end side of the spiral tube 70 are adhered to a distal end side of the inner tube 54 by an adhesive 78. As a result, the spiral tube 70 is firmly adhered to the inner tube 54. It should be noted that, since the spiral tube 70 has the flexibility in the bending direction, the shape thereof is deformable into, for example, the bent shape as shown in Fig. 4.

Returning to Fig. 2, the shape deformable body 58 includes a sheet material 80 that can come into contact with the high friction surface 74 of the spiral tube 70. The sheet material 80 is formed in a cylindrical shape and is arranged along the direction of the axis Ax. As a result, the high friction surface 74 of the spiral tube 70 is covered with the sheet material 80. Further, the sheet material 80 has the flexibility to be deformable along the shape of the tube main body 50 and is made of, for example, a resin such as urethane, but the present invention is not limited to this. In a case where the thickness of the tube main body 50 is, for example, about 1 mm, it is preferable that a thickness of the sheet material 80 is preferably 300 µm or less.

The sheet material 80 includes a high friction surface 82 on an inner peripheral surface that is in contact with the high friction surface 74 of the spiral tube 70. Accordingly, in a case where the air in the inner space 56 is exhausted (sucked) by a vacuum pump 40 (see Figs. 1 and 2) and the inner space 56 is in a decompressed state (for example, a vacuum state), in the tube main body 50, the spiral tube 70 and the sheet material 80 are closely attached to and frictionally engaged with each other via the respective high friction surfaces 74 and 82. As a result, since the shape of the spiral tube 70 is held not to be deformable, the hardness of the tube main body 50 is changed from a soft state to a hard state, and the shape holding force of the tube main body 50 is enhanced. Further, the frictional engagement is released by opening the inner space 56 to the atmosphere and supplying air to the inner space 56, so that the hardness of the tube main body 50 is changed from the hard state to the soft state. The tube main body 50 according to the present example is an example of a tube main body according to the embodiment of the present invention. Further, the outer tube 52, the inner tube 54, and the shape deformable body 58 arranged along the longitudinal direction (axis Ax) of the tube main body 50 are portions of which the hardness is changed as described above, and are examples of a hardness change part according to the embodiment of the present invention.

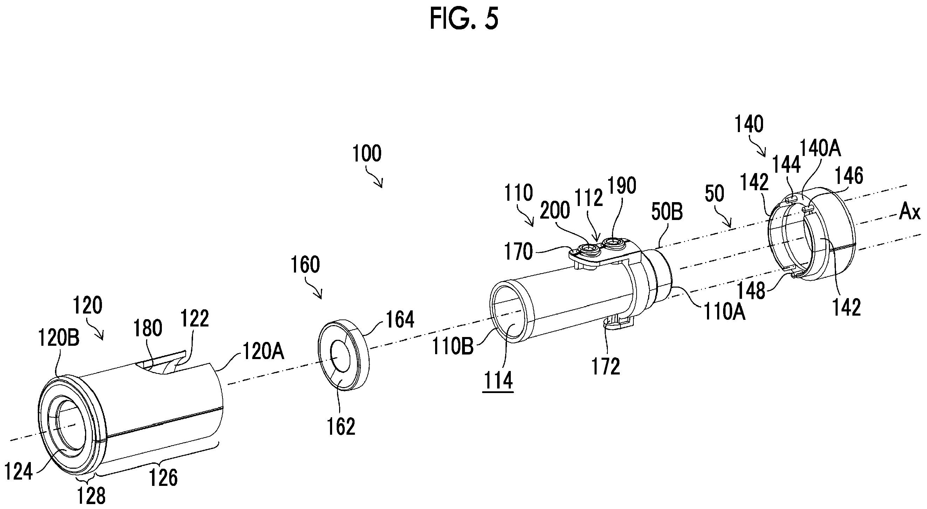

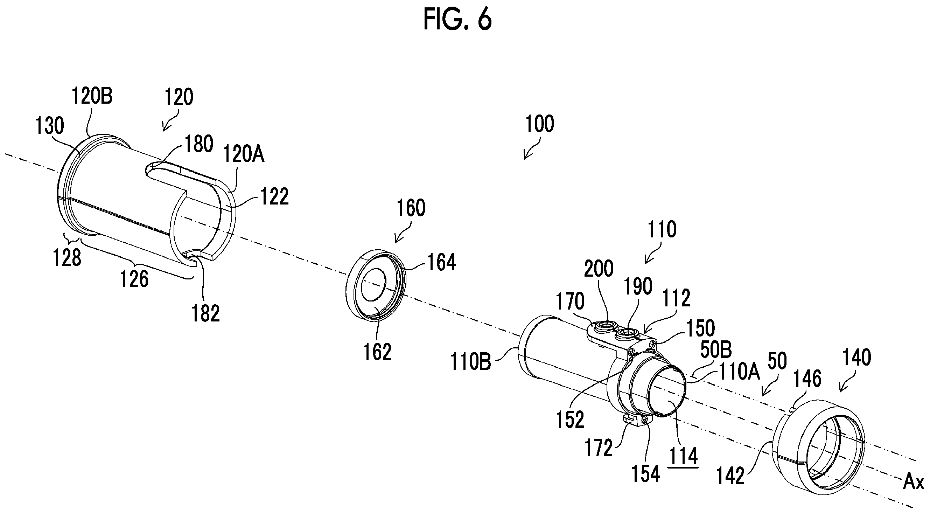

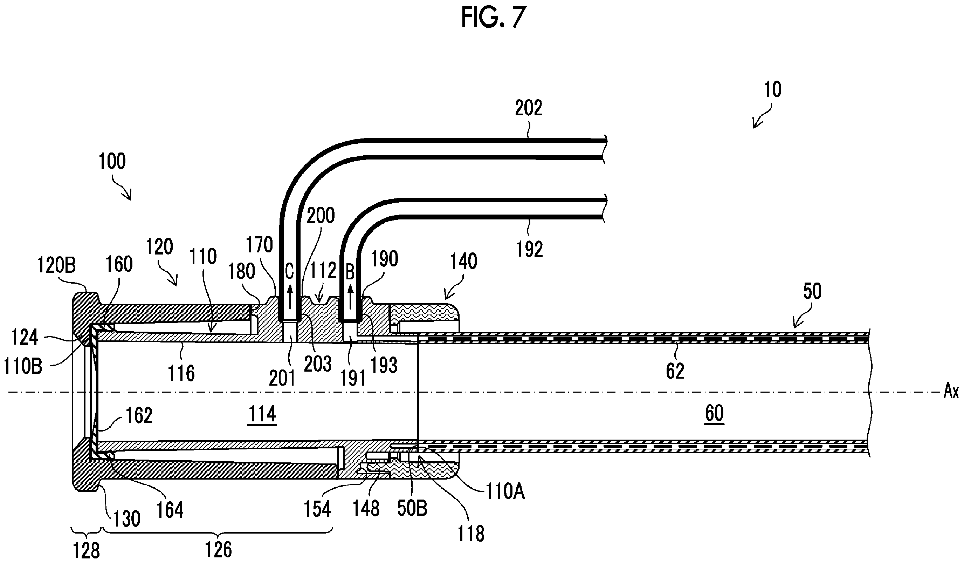

Next, a configuration of the handle part 100 shown in Fig. 1 will be specifically described with reference to Figs. 5 to 7. Fig. 5 is an assembly perspective view in which each member of the handle part 100 is viewed from an oblique direction of a proximal end side (the left side in Fig. 5) of the axis Ax, and Fig. 6 is an assembly perspective view in which each member of the handle part 100 is viewed in an oblique direction on a distal end side (the right side of Fig. 6) of the axis Ax. Fig. 7 is a cross-sectional view of the handle part 100 taken along the axis Ax.

As shown in Figs. 5 to 7, the handle part 100 is provided on the proximal end side of the tube main body 50, and includes the inner sheath 110 to which the proximal end part 50B of the tube main body 50 is connected, and a protection sheath 120 externally inserted into the inner sheath 110. In addition, the inner sheath 110 includes a port part 112 for supplying and discharging a fluid to and from the tube main body 50. The handle part 100 is an example of a handle part according to the embodiment of the present invention. It should be noted that each axis of the inner sheath 110 and the protection sheath 120 is arranged along the axis Ax.

The inner sheath 110 has an outer diameter that is substantially equal to an outer diameter of the tube main body 50. In addition, an insertion passage 114 of the insertion part 14 (see Fig. 1) is defined by an inner peripheral surface of the inner sheath 110. Therefore, the insertion part 14 (see Fig. 1) is inserted into the insertion passages 114 and 60 (see Fig. 2) defined by the inner peripheral surface of the inner sheath 110 and the inner peripheral surface of the inner tube 54 (see Fig. 2), respectively, and guided into the body. It should be noted that a hydrophilic coating 116 is also formed on the inner peripheral surface of the inner sheath 110 as in the inner tube 54, and thus a frictional resistance between the inner sheath 110 and the insertion part 14 is reduced. The inner sheath 110 is an example of an inner sheath according to the embodiment of the present invention.

The protection sheath 120 has an inner diameter that is larger than the outer diameter of the inner sheath 110. As a result, the protection sheath 120 is formed to be externally insertable into the inner sheath 110. The protection sheath 120 is a member that is gripped by operator's fingers, and the outer diameter and the length of the protection sheath 120 in the direction of the axis Ax are formed in the size and the length enough to be gripped by the operator's fingers. For example, the outer diameter of the protection sheath 120 is 3 to 4 cm, and the length of the protection sheath 120 in the direction of the axis Ax is about 8 to 10 cm. The protection sheath 120 is an example of a protection sheath according to the embodiment of the present invention. It should be noted that other configurations of the protection sheath 120 will be described later.

As shown in Fig. 7, in a connection portion 118 of the inner sheath 110 and the tube main body 50, a ring member 140 that surrounds an outer periphery of the connection portion 118 in a loosely fitted state is arranged. The connection portion 118 is a portion on which stress is concentrated and applied in a case where the tube main body 50 is operated to be bent, and includes, for example, a length portion of about 2 cm from the proximal end part 50B of the tube main body 50 toward the distal end side in the direction of the axis Ax (hereinafter, also referred to as a "proximal end side portion of the tube main body 50"), in addition to the proximal end part 50B of the tube main body 50 and a coating region with the adhesive 68 (see Fig. 2). In a case where the tube main body 50 is operated to be bent, the proximal end side portion of the tube main body 50 comes into contact with the inner peripheral surface of the ring member 140, so that an excessive bending operation of the proximal end side portion of the tube main body 50 is restricted. Therefore, the ring member 140 has a function of relaxing the stress that is generated in the connection portion 118 in a case where the tube main body 50 is operated to be bent. The ring member 140 is an example of a stress relaxation ring according to the embodiment of the present invention.

Here, the guide tube 10 according to the present example adopts a configuration in which the inner sheath 110 and the protection sheath 120 are coupled and fixed to the ring member 140. Hereinafter, an example of the configuration for coupling and fixing the inner sheath 110 and the protection sheath 120 to the ring member 140 will be described.

As shown in Fig. 5, the ring member 140 includes a pair of arc-shaped protrusions 142 on a proximal end surface 140A of the ring member 140, and these protrusions 142 are provided to protrude from the proximal end surface 140A toward the proximal end side in the direction of the axis Ax. Further, these protrusions 142 are provided along a circumference centered on an axis (the axis Ax after the coupling and fixing) of the ring member 140, and the protection sheath 120 is coupled and fixed to the ring member 140 by externally fitting a proximal end side opening part 122 of the protection sheath 120 onto these protrusions 142.

Further, as shown in Fig. 5, the ring member 140 includes three pins 144, 146, and 148 on the proximal end surface 140A, and these pins 144, 146, and 148 are provided to protrude from the proximal end surface 140A toward the proximal end side in the direction of the axis Ax. Among the pins 144, 146, and 148, the pins 144 and 146 are provided to be adjacent to each other, and the pin 148 is provided at a position spaced from the pins 144 and 146 by substantially 180 degrees in a circumferential direction. The three pins 144, 146, and 148 are fitted into three holes 150, 152, and 154 provided on the proximal end side of the inner sheath 110 shown in Fig. 6. In other words, the pin 144 is fitted into the hole 150, the pin 146 is fitted into the hole 152, and the pin 148 is fitted into the hole 154. The three pins 144, 146, and 148 are fitted into the three holes 150, 152, and 154 in this way, so that the inner sheath 110 is coupled and fixed to the ring member 140. As a result, the inner sheath 110 and the protection sheath 120 are coupled and fixed to the ring member 140. The configuration described above is an example of a configuration for coupling and fixing the inner sheath 110 and the protection sheath 120 to the ring member 140. It should be noted that it is preferable that the protection sheath 120 and the ring member 140 are fixed to each other by an adhesive (not shown) after the coupling and fixing described above.

As shown in Fig. 7, a leakage prevention valve 160 (see Figs. 5 and 6) is arranged inside the handle part 100. The leakage prevention valve 160 has a function of preventing a liquid from leaking from the tube main body 50 by coming into close contact with the outer peripheral surface of the insertion part 14 in a case where the insertion part 14 (see Fig. 1) is inserted into the handle part 100. Hereinafter, an example of the configuration for arranging the leakage prevention valve 160 inside the handle part 100 will be described.

As shown in Fig. 7, the protection sheath 120 includes a flange-shaped valve pressing part 124 provided on the inner peripheral surface of the protection sheath 120. The leakage prevention valve 160 is interposed between the valve pressing part 124 and a proximal end part 110B of the inner sheath 110, and the position of the handle part 100 in the direction of the axis Ax is restricted.

A specific arrangement configuration of the leakage prevention valve 160 will be described. As shown in Figs. 5 to 7, the leakage prevention valve 160 is formed in a substantially ring shape, and includes an annular valve body part 162, a tubular part 164 provided to protrude from an outer peripheral part of the valve body part 162 toward the distal end side in the direction of the axis Ax. An outer diameter of the tubular part 164 is smaller than the inner diameter of the protection sheath 120, and an inner diameter of the tubular part 164 is larger than the outer diameter of the inner sheath 110. Further, an inner diameter of the valve body part 162 is smaller than the inner diameter of the inner sheath 110 and is smaller than the outer diameter of the insertion part 14 (see Fig. 1). The valve pressing part 124 is provided on an inner peripheral surface of a proximal end part 120B of the protection sheath 120, and an inner diameter of the valve pressing part 124 is larger than the inner diameter of the valve body part 162 and is smaller than the inner diameter of the inner sheath 110. With such an arrangement configuration of the leakage prevention valve 160, on the proximal end part 120B of the protection sheath 120, the leakage prevention valve 160 is arranged with the valve body part 162 interposed between the valve pressing part 124 and the proximal end part 110B of the inner sheath 110. The leakage prevention valve 160 is an example of a leakage prevention valve according to the embodiment of the present invention, and the valve pressing part 124 is an example of a valve pressing part according to the embodiment of the present invention.

It should be noted that the arrangement configuration described above is a configuration in which the valve pressing part 124 is provided on the proximal end part 120B of the protection sheath 120, but the present invention is not limited to this, and the valve pressing part 124 may be provided at a position spaced from the proximal end part 120B to the distal end side in the direction of the axis Ax. However, from the viewpoint of stably restricting the position of the leakage prevention valve 160 in the direction of the axis Ax, the above-described arrangement configuration in which the valve pressing part 124 is provided on the proximal end part 120B of the protection sheath 120 is preferable.

As shown in Figs. 5 and 6, the inner sheath 110 includes a base part 170 provided to protrude on an outer peripheral surface of the inner sheath 110. In addition, the protection sheath 120 includes a slit 180 open toward the tube main body 50 side. As shown in Fig. 7, the protection sheath 120 is externally inserted into the inner sheath 110 in a state in which rotation of the protection sheath 120 is locked with respect to the inner sheath 110 by engaging the slit 180 and the base part 170 with each other. Hereinafter, an example of the configuration for engaging the base part 170 and the slit 180 with each other will be described.

As shown in Figs. 5 and 6, the base part 170 is formed as a substantially rectangular plate-shaped part that extends from the distal end part 110A side of the inner sheath 110 toward the proximal end side in the direction of the axis Ax. Further, the slit 180 is formed as a substantially rectangular notch part that is formed from the distal end part 120A of the protection sheath 120 toward the proximal end side in the direction of the axis Ax, and has a length in the direction of the axis Ax and a width in the circumferential direction about the axis Ax which are substantially the same length and width as the base part 170. Due to this configuration, the slit 180 and the base part 170 are engaged with each other. As a result, the protection sheath 120 is externally inserted into the inner sheath 110 in a state in which the rotation of the protection sheath 120 is locked in the circumferential direction about the axis Ax with respect to the inner sheath 110. The base part 170 is arranged to be exposed on an outer surface of the protection sheath 120 (see Fig. 1). The base part 170 is an example of an engaging part according to the embodiment of the present invention, and the slit 180 is an example of an engaged part according to the embodiment of the present invention. It should be noted that, as shown in Fig. 6, the holes 150 and 152 into which the pins 144 and 146 (see Fig. 5) are fitted are provided in a distal end side surface of the base part 170. Further, the hole 154 into which the pin 148 (see Fig. 5) is fitted is provided in a distal end side surface of a protrusion part 172 provided to protrude from the outer peripheral surface of the inner sheath 110. The protrusion part 172 is engaged with a notch part 182 formed on the distal end part 120A of the protection sheath 120 during the external insertion.

As shown in Fig. 5 to Fig. 7, the port part 112 is provided in the base part 170. As shown in Fig. 7, the port part 112 includes a port 190 for supplying and discharging the fluid (for example, air) to and from the tube main body 50 and a port 200 for supplying the liquid (for example, water) to the inner peripheral surface of the insertion passage 114 (inner peripheral surface of the inner sheath 110). The port part 112, the port 190, and the port 200 are examples of a port part, a fluid port, and a liquid port according to the embodiment of the present invention, respectively. Further, the port 190 communicates with the inner space 56 (see Fig. 2) of the tube main body 50 via a flow passage 191 formed in the inner sheath 110, and the port 200 communicates with the inner peripheral surface of the inner sheath 110 via a flow passage 201 formed in the inner sheath 110.

As shown in Figs. 5 to 7, the port 190 and the port 200 are installed adjacent to each other along the direction of the axis Ax of the inner sheath 110. In other words, the port 190 and the port 200 are installed adjacent to each other along the same axis parallel to the axis Ax. In addition, the port 200 is arranged on a side opposite to a side of the inner sheath 110 to which the tube main body 50 is connected, with respect to the port 190. In other words, the port 200 is arranged on the proximal end side in the direction of the axis Ax with respect to the port 190. A tube 192 is connected to the port 190, and a tube 202 is connected to the port 200.

As shown in Fig. 7, a pull-out direction B of a proximal end side portion 193 of the tube 192 from the port 190 and a pull-out direction C of a proximal end side portion 203 of the tube 202 from the port 200 are set to be the same direction. In the configuration of the present example, both the pull-out directions B and C are set to be directions perpendicular to the direction of the axis Ax of the inner sheath 110. The proximal end side portion 193 of the tube 192 includes an insertion portion that is inserted (fitted) into the port 190 that is a hole, and the proximal end side portion 203 of the tube 202 includes an insertion portion that is inserted (fitted) into the port 200 that is a hole. The tube 192 is an example of a fluid tube according to the embodiment of the present invention, and the tube 202 is an example of a liquid tube according to the embodiment of the present invention. It should be noted that, with the configuration in which the hardness of the proximal end side portions 193 and 203 of the tubes 192 and 202 is higher than the hardness of the other portions except for the proximal end side portions 193 and 203, the connection work of the proximal end side portions 193 and 203 to the ports 190 and 200 is facilitated.

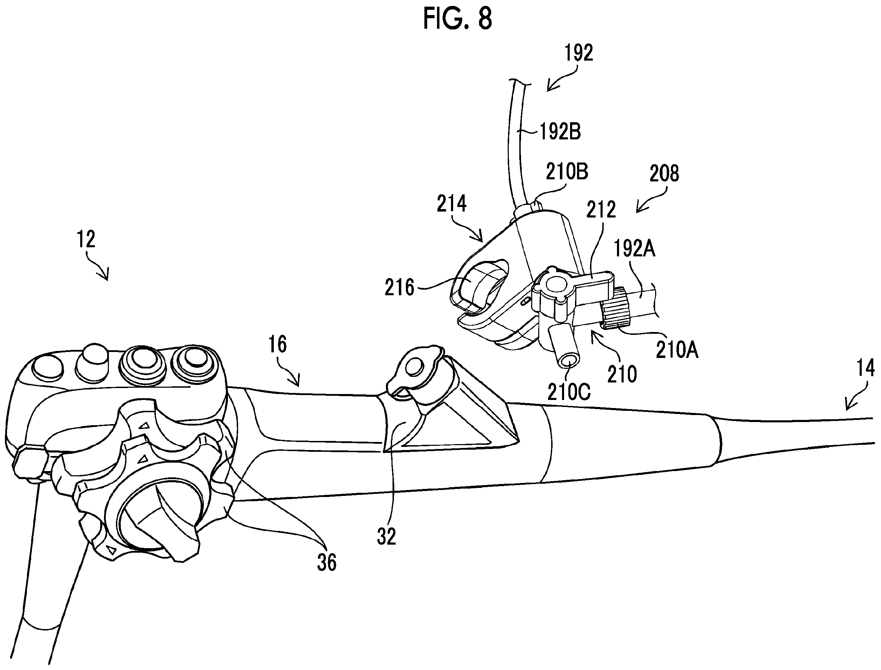

Fig. 8 is a perspective view of a switching unit 208 provided in the middle of the tube 192. The switching unit 208 is an example of a switching unit according to the embodiment of the present invention. Further, the switching unit 208 according to the present example includes a three way stopcock 210. Hereinafter, a function of the three way stopcock 210 will be described with reference to a schematic view of the three way stopcock 210 shown in Fig. 2.

As shown in Fig. 2, the three way stopcock 210 includes a first port 210A connected to the tube 192A on the vacuum pump 40 side, a second port 210B connected to the tube 192B on the port 190 side, and a third port 210C for opening to the atmosphere, in the tube 192. Also, the three way stopcock 210 includes a cock 212. By operating the cock 212 to be moved rotationally, the operator can selectively switch between an ON mode in which the first port 210A and the second port 210B communicate with each other and an OFF mode in which the second port 210B and the third port 210C communicate with each other. In a case where the cock 212 is switched to the ON mode, the vacuum pump 40 and the inner space 56 of the tube main body 50 communicate with each other via the tube 192 and the flow passage 191. As a result, the air in the inner space 56 is exhausted (sucked) by the vacuum pump 40, the inner space 56 is in the decompressed state (for example, the vacuum state), and the hardness of the tube main body 50 is in the hard state. In a case where the cock 212 is switched to the OFF mode, the inner space 56 and outside air communicate with each other via the tube 192 and the flow passage 191. As a result, air (outside air) is supplied from the tube 192 to the inner space 56 via the flow passage 191, the inner space 56 is in an atmospheric pressure state, and the hardness of the tube main body 50 is in the soft state. In a case where the cock 212 of the three way stopcock 210 is operated in this way, the supply and discharge of the air to and from the inner space 56 can be switched. The three way stopcock 210 is an example of a switching member according to the embodiment of the present invention.

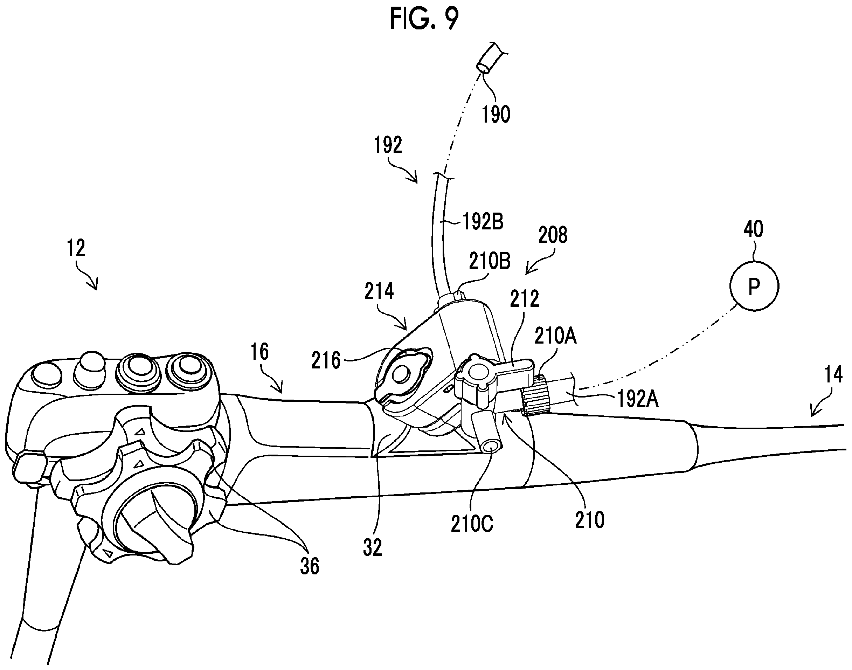

Returning to Fig. 8, the switching unit 208 includes a hook 214 attachably and detachably fixable to the endoscope 12. The hook 214 includes an opening part 216 that can be fitted into the treatment tool insertion part 32 of the endoscope 12. As shown in Fig. 9, the opening part 216 of the hook 214 is fitted into the treatment tool insertion part 32, so that the hook 214 is fixed to the treatment tool insertion part 32 and the switching unit 208 is arranged on the hand operating part 16 (see Fig. 1). As a result, the operator who operates the hand operating part 16 can operate the cock 212 of the three way stopcock 210. The hook 214 is an example of a fixing part according to the embodiment of the present invention, and the opening part 216 is an example of a fitting part according to the embodiment of the present invention.

As shown in Fig. 9, the cock 212 of the three way stopcock 210 is arranged on a side opposite to the port 190 side with respect to the hook 214. In other words, the cock 212 of the three way stopcock 210 is arranged on the vacuum pump 40 side with respect to the hook 214. As a result, the operator can operate the cock 212 with the right hand while gripping the hand operating part 16 with the left hand.

It should be noted that, although the tube 192 (including the tube 192A and the tube 192B) has a length (for example, about 3 to 5 meters) required for connecting the port 190 and the vacuum pump 40, the tube 192B need only have a length (for example, about 1 meter) for arranging the switching unit 208 on the hand operating part 16. In this case, the switching unit 208 is provided in the tube 192 at an intermediate position spaced from the port 190 by about 1 meter. The switching unit 208 is provided at such an intermediate position, so that the operator can operate the cock 212 of the three way stopcock 210 without drawing the tube 192B.

Returning to Fig. 1, a syringe 204 is connected to a distal end part 202A of the tube 202. The liquid (for example, water) is stored in the syringe 204, and the liquid can be supplied from the tube 202 to the inner peripheral surface of the inner sheath 110 via the flow passage 201 of Fig. 7 by the operator operating the syringe. As a result, the hydrophilic coating 116 formed on the inner peripheral surface of the inner sheath 110 can be wetted with the liquid. It should be noted that, in order to make the syringe 204 that can be operated by the operator together with the three way stopcock 210, the tube 202 need only have, for example, a length (for example, about 1 meter) that is substantially equal to the length of the tube 192B. As a result, the operator can operate the syringe 204 without drawing the tube 202.

Next, a configuration of the protection sheath 120 will be described. As shown in Figs. 5 to 7, the protection sheath 120 includes a first sleeve part 126 and a second sleeve part 128. The first sleeve part 126 and the second sleeve part 128 are arranged along the direction of the axis Ax, the first sleeve part 126 is arranged on the tube main body 50 side, and the second sleeve part 128 is arranged on a side opposite to the tube main body 50 side with respect to the first sleeve part 126. In other words, the second sleeve part 128 is arranged on the proximal end side in the direction of the axis Ax with respect to the first sleeve part 126. Further, the second sleeve part 128 is formed to have a larger outer diameter than the first sleeve part 126.

The first sleeve part 126 is a portion that forms a body part of the protection sheath 120, that is, the first sleeve part 126 functions as a portion that is formed to be longer than the second sleeve part 128 in the direction of the axis Ax and is gripped by the operator's fingers. The first sleeve part 126 is an example of a first sleeve part according to the embodiment of the present invention.

The second sleeve part 128 is formed in a flange shape on the proximal end part 120B of the protection sheath 120. The second sleeve part 128 is provided mainly to form a step part 130 between the second sleeve part 128 and the first sleeve part 126, and the operator can use this step part 130 as a finger hook part for hooking the fingers. The second sleeve part 128 is an example of a second sleeve part according to the embodiment of the present invention.

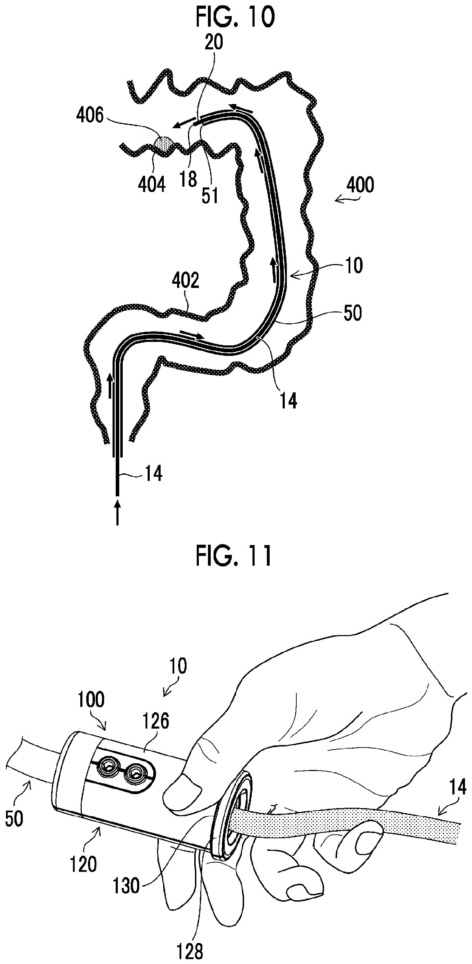

Next, an example of an operation in a case where the insertion part 14 of the endoscope 12 is guided to a large intestine 400 (see Fig. 10) by using the guide tube 10 will be described.

First, since the insertion part 14 and the tube main body 50 each have the flexibility, in a case where the insertion part 14 (see Fig. 1) inserted into the insertion passages 114 and 60 (see Fig. 7) of the guide tube 10 is inserted along the large intestine 400 (see Fig. 10) together with the tube main body 50, the insertion part 14 and the tube main body 50 are smoothly inserted along a bent shape of the large intestine 400.

Next, in a state in which the tube main body 50 is arranged along a bent shape of a sigmoid colon 402, the cock 212 of the three way stopcock 210 is operated to switch to the ON mode. Then, the air in the inner space 56 is exhausted (sucked) by the vacuum pump 40 (see Fig. 1), the sheet material 80 is pressed against the outer tube 52, and the spiral tube 70 is pressed against the inner tube 54. As a result, the high friction surface 74 of the spiral tube 70 and the high friction surface 82 of the sheet material 80 are closely attached to and frictionally engaged with each other. As a result, the tube main body 50 is hardened in a shape corresponding to the bent shape of the sigmoid colon 402, and the shape is held, so that the insertability of the insertion part 14 into the sigmoid colon 402 is improved.

Next, the treatment of a lesion portion 406 of a transverse colon 404 is started by causing the bendable part 20 of the insertion part 14 to protrude forward from the distal end opening part 51 of the guide tube 10 and then leading out the treatment tool (not shown) forward from the treatment tool outlet port 28 (see Fig. 1) of the distal end hard part 18. In this case, since the insertability of the insertion part 14 into the sigmoid colon 402 is improved by the guide tube 10, the distal end hard part 18 can be positioned at an accurate treatment position, and as a result, it is possible to perform an accurate treatment on the lesion portion 406.

During the above-described treatment of the endoscope 12, the operator inserts the tube main body 50 of the guide tube 10 and the insertion part 14 into the body while generally gripping the hand operating part 16 with the left hand and gripping the handle part 100 of the guide tube 10 and the insertion part 14 with the right hand. Then, in a case where the hardness of the tube main body 50 is changed, the cock 212 of the three way stopcock 210 is operated with the right hand. Then, in a case where the treatment for the lesion portion 406 is finished, the handle part 100 and the insertion part 14 are similarly gripped with the right hand to pull out the tube main body 50 and the insertion part 14 from the inside of the body.

It should be noted that, in the operation method described with reference to Fig. 10, the tube main body 50 (including the insertion part 14) in the soft state is hardened in a state along the bent shape of the sigmoid colon 402, but the present invention is not limited to this operation method. For example, an operation method may be adopted in which the sigmoid colon 402 is linearized with the tube main body 50 (including the insertion part 14) in the soft state and then the tube main body 50 is hardened. As the linearization of the sigmoid colon 402, as an example, the tube main body 50 (including the insertion part 14) in the soft state is inserted along the bent shape of the sigmoid colon 402, and in a case where the distal end of the tube main body 50 reaches a spleen portion (not shown), the tube main body 50 is operated in the pulling-out direction while being rotated about the axis Ax. With such an operation, the sigmoid colon 402 can be linearized. Also, an operation method may be adopted in which the tube main body 50 is inserted into the intestinal tract after the intestinal tract is linearized to a certain extent by the insertion part 14, and then the tube main body 50 is hardened.

Hereinafter, effects of the guide tube 10 according to the first embodiment will be described.

With the guide tube 10, since the simple configuration is adopted in which the handle part 100 includes the inner sheath 110 to which the proximal end part 50B of the tube main body 50 is connected and that includes the port part 112 for supplying and discharging the fluid to and from the tube main body 50, and the protection sheath 120 externally inserted into the inner sheath 110, the handle part 100 is easily gripped by the operator who operates the guide tube 10. As a result, with the guide tube 10 according to the first embodiment, it is possible to improve the operability of the endoscope 12 that is operated by using the guide tube 10.

In addition, since the guide tube 10 adopts the configuration in which the ring member 140 that surrounds the outer periphery of the connection portion 118 between the inner sheath 110 and the tube main body 50 in the loosely fitted state is arranged, the stress generated in the connection portion 118 in a case where the tube main body 50 is bent can be relaxed by the ring member 140. In other words, in a case where the proximal end side portion of the tube main body 50 is bent during the bending operation of the tube main body 50, a part of the proximal end side portion of the tube main body 50 comes into contact with the inner peripheral surface of the ring member 140, so that an excessive bending operation of the proximal end side portion of the tube main body 50 is restricted by the ring member 140, the stress generated in the connection portion 118 can be relaxed, and the connection portion 118 can be protected. Further, since the configuration is adopted in which the handle part 100 is assembled by coupling and fixing the inner sheath 110 and the protection sheath 120 to the ring member 140, the ease of assembly and fastness of the handle part 100 are improved.

In addition, since the guide tube 10 adopts the configuration in which the leakage prevention valve 160 is arranged inside the handle part 100, for example, the leakage prevention valve 160 can be arranged in the handle part 100 with a simpler configuration than a configuration in which the leakage prevention valve 160 is externally attached to the outside of the handle part 100 by using a cap. As a result, the ease of assembly of the handle part 100 is improved.

Further, with the guide tube 10, since the configuration is adopted in which the position of the leakage prevention valve 160 in the direction of the axis Ax is restricted by interposing the leakage prevention valve 160 between the flange-shaped valve pressing part 124 provided on the inner peripheral surface of the protection sheath 120 and the proximal end part 110B of the inner sheath 110, it is possible to restrict the misregistration of the leakage prevention valve 160 in the direction of the axis Ax with a simple configuration. In addition, with the configuration of the present example, it is also possible to restrict the misregistration of the leakage prevention valve 160 in the direction perpendicular to the axis Ax. Therefore, with the configuration of the present example, it is possible to effectively prevent the liquid from leaking.

Further, since the guide tube 10 adopts the configuration in which the protection sheath 120 is externally inserted into the inner sheath 110 in a state in which the rotation of the protection sheath 120 is locked with respect to the inner sheath 110 by engaging the base part 170 with the slit 180, it is possible to realize the lock of the rotation of the protection sheath 120 with a simple configuration. As a result, the ease of assembly of the handle part 100 is improved.

In addition, since the guide tube 10 adopts the configuration in which the port part 112 is provided at the base part 170, that is, the configuration in which the port part 112 is provided at the base part 170 exposed to the outside of the handle part 100, the connection work of connecting the tube 192 for supplying and discharging the fluid to the port part 112 is facilitated.

Further, with the guide tube 10, since the configuration is adopted in which the port part 112 includes the port 190 and the port 200, the connection work of connecting the tube 192 to the port 190 and the connection work of connecting the tube 202 to the port 200 are facilitated.

Furthermore, with the guide tube 10, since the configuration is adopted in which the port 190 and the port 200 are installed adjacent to each other along the direction of the axis Ax of the inner sheath 110, the operator can grip the handle part 100 without being obstructed by the tube 192 and the tube 202. As a result, the operability of the guide tube 10 and the endoscope 12 is improved. It should be noted that, in a case where the port 190 and the port 200 are provided at positions shifted from each other in the direction of the axis Ax in the configuration of the present example, the tube 192 and the tube 202 may obstruct the operator who grips the handle part 100. However, with the configuration of the present example, such a problem can be eliminated.

Furthermore, with the guide tube 10, since the configuration is adopted in which the port 200 is arranged on the side opposite to the side of the inner sheath 110 to which the tube main body 50 is connected, with respect to the port 190, the flow passage 191 from the port 190 toward the tube main body 50 and the flow passage 201 from the port 200 toward the insertion passage 114 can be arranged without intersecting each other. In other words, as shown in Fig. 7, since the flow passage 201 need only be arranged in the direction perpendicular to the direction of the axis Ax from the port 200 toward the insertion passage 114, and the flow passage 191 need only be arranged in an L-shape from the port 190 toward the tube main body 50, both flow passages 191 and 201 can be arranged without intersecting each other. Therefore, by adopting the configuration of the present example, the two flow passages 191 and 201 can be arranged on the inner sheath 110 with a simple configuration.

Furthermore, with the guide tube 10, since the configuration is adopted in which the pull-out direction B of the proximal end side portion 193 of the tube 192 from the port 190 and the pull-out direction C of the proximal end side portion 203 of the tube 202 from the port 200 are set to be the same direction, the operator can grip the handle part 100 without being obstructed by the tube 192 and the tube 202. As a result, the operability of the guide tube 10 and the endoscope 12 is improved. It should be noted that, for example, in a case where both the pull-out directions B and C are different from each other (for example, the pull-out direction B is set to the distal end side in the direction of the axis Ax, and the pull-out direction C is set to the proximal end side in the direction of the axis Ax) in the configuration of the present example, the tube 192 and the tube 202 may obstruct the operator who grips the handle part 100. However, with the configuration of the present example, such a problem can be eliminated.

Furthermore, with the guide tube 10, since the configuration is adopted in which the pull-out directions B and C are the directions perpendicular to the direction of the axis Ax of the inner sheath 110, it is possible for the operator who views the handle part 100 to easily visually recognize, in terms of appearance, a boundary between a handle area (outer surface of the protection sheath 120) gripped by the operator and a tube area (outer surface of the base part 170) to which the tube 192 and the tube 202 are connected. As a result, the operator can avoid the tube area and grip the handle area only by viewing the handle part 100 at a glance. As a result, the operability of the guide tube 10 and the endoscope 12 is improved.

Further, with the guide tube 10, since the configuration is adopted in which the protection sheath 120 includes the first sleeve part 126 arranged on the tube main body 50 side and the second sleeve part 128 arranged on the side opposite to the tube main body 50 side with respect to the first sleeve part 126 and having a larger outer diameter than the first sleeve part 126, as shown in Fig. 11, it is possible to use the step part 130 formed between the first sleeve part 126 and the second sleeve part 128 as the finger hook part for hooking the operator's fingers. As a result, since the operator can perform an operation of inserting and pulling out the insertion part 14 into and from the guide tube 10 or pulling out the guide tube 10 itself from the inside of the body in a state in which the fingers are hooked to the step part 130 of the guide tube 10, the operability of the guide tube 10 and the endoscope 12 is improved. It should be noted that Fig. 11 shows an operation example of the endoscope 12 using the guide tube 10, but the tubes 192 and 202 (see Fig. 7) are not shown.

Further, since the guide tube 10 adopts the configuration in which the switching unit 208 is provided in the middle of the tube 192, the operator can operate the cock 212 of the three way stopcock 210 without drawing the tube 192B. As a result, the operability of the guide tube 10 and the endoscope 12 is improved.

Further, with the guide tube 10, since the configuration is adopted in which the switching unit 208 includes the hook 214 attachably and detachably fixable to the endoscope 12, the switching unit 208 can be fixed to the endoscope 12 by using the hook 214. As a result, since the operator can operate the cock 212 of the three way stopcock 210 while operating the endoscope 12, the operability of the guide tube 10 and the endoscope 12 is improved.

Furthermore, with the guide tube 10, since the configuration is adopted in which the hook 214 includes the opening part 216 that can be fitted into the treatment tool insertion part 32 of the endoscope 12, the switching unit 208 can be fixed to the endoscope 12 by only fitting the opening part 216 of the hook 214 into the treatment tool insertion part 32.

Furthermore, with the guide tube 10, since the configuration is adopted in which the three way stopcock 210 is arranged on the side opposite to the port 190 side with respect to the hook 214 in a case where the switching unit 208 is attachably and detachably fixed to the endoscope 12, the operator can, for example, operate the cock 212 of the three way stopcock 210 with the left hand while gripping the hand operating part 16 with the left hand and operating the endoscope 12 (for example, operating the bendable part 20 to be bent). As a result, the operability of the guide tube 10 and the endoscope 12 is improved.

### Second embodiment

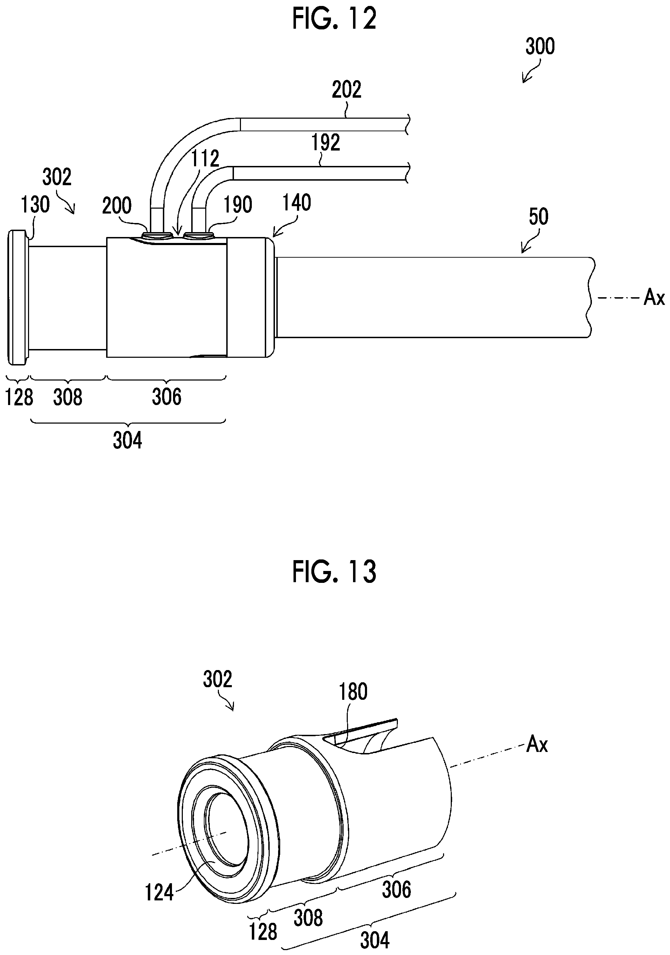

Fig. 12 is an external view showing a main part of a guide tube 300 according to a second embodiment. In a case where the guide tube 300 according to the second embodiment is described, the same members as or members similar to the members of the guide tube 10 according to the first embodiment shown in Figs. 1 to 11 will be denoted by the same reference numerals.

The difference in configuration between the first embodiment and the second embodiment is that the first sleeve part 126 of the protection sheath 120 (see Figs. 5 to 7) according to the first embodiment has the same outer diameter over the entire direction of the axis Ax, whereas a first sleeve part 304 of a protection sheath 302 according to the second embodiment includes a sleeve body large-diameter part 306 and a sleeve body small-diameter part 308 which have different outer diameters in the direction of the axis Ax. Since other configurations are the same, a protection sheath 302 according to the second embodiment will be described here.

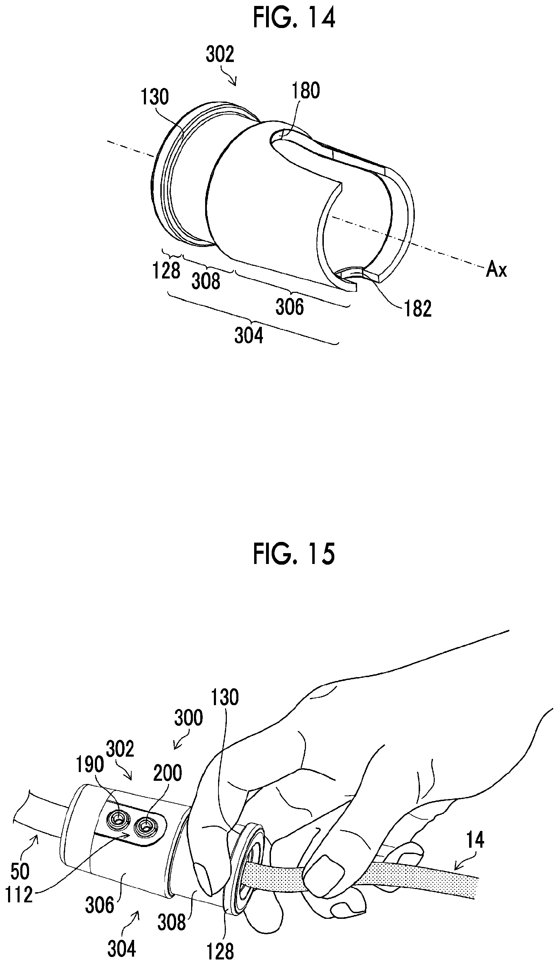

Fig. 13 is a perspective view of the protection sheath 302 as viewed from an oblique direction on a proximal end side (the left side in Fig. 13) of the axis Ax, and Fig. 14 is a perspective view of the protection sheath 302 as viewed from an oblique direction on a distal end side (the right side in Fig. 14) of the axis Ax.

As shown in Figs. 12 to 14, the first sleeve part 304 includes the sleeve body large-diameter part 306 arranged on the tube main body 50 side, and the sleeve body small-diameter part 308 arranged on a side opposite to the tube main body 50 side with respect to the sleeve body large-diameter part 306. In other words, the sleeve body small-diameter part 308 is arranged on the proximal end side in the direction of the axis Ax with respect to the sleeve body large-diameter part 306. Further, the sleeve body small-diameter part 308 is formed to have a smaller outer diameter than the sleeve body large-diameter part 306 and the second sleeve part 128, and is arranged on the distal end side in the direction of the axis Ax with respect to the second sleeve part 128. Further, the sleeve body small-diameter part 308 is formed such that a length in the direction of the axis Ax of the sleeve body small-diameter part 308 is longer than a length in the direction of the axis Ax of the second sleeve part 128. Furthermore, the sleeve body small-diameter part 308 is arranged on a side opposite to the tube main body 50 side with respect to the port part 112. In other words, the sleeve body small-diameter part 308 is arranged on the proximal end side in the direction of the axis Ax with respect to the port part 112.

In the protection sheath 302 according to the present example, since the first sleeve part 304 includes the sleeve body large-diameter part 306 arranged on the tube main body 50 side, and the sleeve body small-diameter part 308 arranged on a side opposite to the tube main body 50 side with respect to the sleeve body large-diameter part 306, as shown in Fig. 15, the sleeve body small-diameter part 308 formed between the sleeve body large-diameter part 306 and the second sleeve part 128 can be used as a finger insertion part into which the operator's fingers are inserted. As a result, the operator can perform the operation of inserting, for example, the index finger and the middle finger into the sleeve body small-diameter part 308 of the guide tube 300, inserting and pulling out the insertion part 14 into and from the guide tube 300 in a state where the sleeve body small-diameter part 308 is pinched with the index finger and the middle finger, and or pulling out the guide tube 300 itself from the inside of the body. As a result, with the guide tube 300 according to the second embodiment, the operability of the endoscope 12 can be improved. It should be noted that Fig. 15 shows an operation example of the endoscope 12 using the guide tube 300, but the tubes 192 and 202 (see Fig. 7) are not shown.

Further, Fig. 15 shows an example in which the sleeve body small-diameter part 308 is pinched by the index finger and the middle finger, but the present invention is not limited to this. For example, the operation of inserting and pulling out the guide tube 300 may be performed in a state in which the operator's index finger is hooked along an outer peripheral surface of the sleeve body small-diameter part 308.

Further, since the protection sheath 302 according to the present example is formed such that the length in the direction of the axis Ax of the sleeve body small-diameter part 308 is longer than the length in the direction of the axis Ax of the second sleeve part 128, the sleeve body small-diameter part 308 can be effectively used as a portion pinched or gripped by the operator. As a result, a force for the operation of inserting and pulling out the guide tube 300 can be effectively transmitted to the guide tube 300. It should be noted that, since the second sleeve part 128 need only be formed as a sleeve part for forming the step part 130 for hooking the fingers, even in a case where the length in the direction of the axis Ax of the second sleeve part 128 is shorter than the length in the direction of the axis Ax of the sleeve body small-diameter part 308, the function of the guide tube 300 is not hindered.

Further, in the protection sheath 302 according to the present example, as shown in Fig. 12, since the sleeve body small-diameter part 308 is arranged on the side opposite to the tube main body 50 side with respect to the port part 112, the sleeve body small-diameter part 308 can be pinched or gripped with the operator's fingers without being obstructed by the port part 112.

Hereinafter, some modification examples of the present invention will be described.

### First modification example

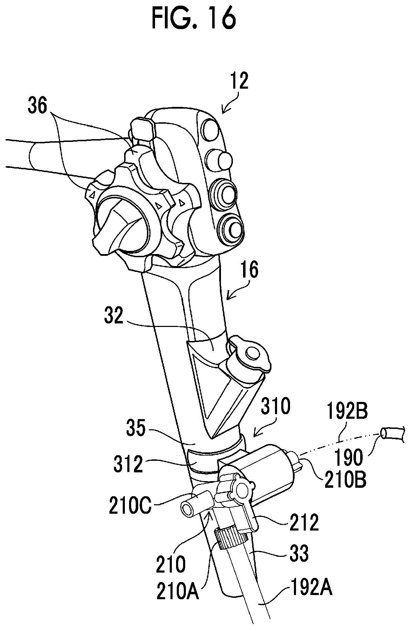

Fig. 16 shows a modification example of a switching unit 310 including the three way stopcock 210. The switching unit 310 is an example of a switching unit according to the embodiment of the present invention.

The switching unit 310 includes a hook 312 bent in a C-shape by an elastic plate material. The hook 312 can be mounted on a cylindrical part 35 positioned between the treatment tool insertion part 32 and a bending prevention part 33 of the hand operating part 16 with one touch. As a result, the switching unit 310 can be fixed to the hand operating part 16 by using the hook 312. The hook 312 is an example of a fixing part according to the embodiment of the present invention. It should be noted that, even in the switching unit 310 according to the present example, since the three way stopcock 210 is arranged on a side opposite to the port 190 side with respect to the hook 312, the operability of the cock 212 of the three way stopcock 210 is improved.

### Second modification example

In the first and second embodiments described above, although the tube main body 50 including the hardness change part is described as the tube main body applied to the medical instrument guide device according to the embodiment of the present invention, the present invention is not limited to this. For example, an overtube for an endoscope comprising a balloon at the distal end part and having a flow passage for supplying and discharging the fluid to and from the balloon may be applied.

### Third modification example

In the first and second embodiments described above, although the endoscope 12 including the insertion part 14 is described as the medical instrument guided by the medical instrument guide device according to the embodiment of the present invention, the present invention is not limited to this. For example, the present invention can be applied to a medical instrument, such as a manipulator.

### Additional note

As understood from the description of the embodiments described above in detail, the present specification includes the disclosure of various technical ideas including the following inventions.

### Invention 1

A medical instrument guide device having an insertion passage for guiding a medical instrument into a body, the medical instrument guide device comprising: a long tube main body to be inserted into the body; and a handle part provided on a proximal end side of the tube main body, in which the handle part includes an inner sheath to which a proximal end part of the tube main body is connected and a protection sheath externally inserted into the inner sheath, and the inner sheath includes a port part for supplying and discharging a fluid to and from the tube main body.

### Invention 2

The medical instrument guide device according to Invention 1, in which the tube main body includes a hardness change part that is arranged along a longitudinal direction of the tube main body and of which hardness is changed due to the fluid supplied and discharged from the port part.

### Invention 3

The medical instrument guide device according to Invention 1 or 2, in which a stress relaxation ring that surrounds an outer periphery of a connection portion between the inner sheath and the tube main body in a loosely fitted state is arranged, and the inner sheath and the protection sheath are coupled and fixed to the stress relaxation ring.

### Invention 4

The medical instrument guide device according to any one of Inventions 1 to 3, in which a leakage prevention valve that prevents a liquid from leaking from the tube main body is arranged inside the handle part.

### Invention 5

The medical instrument guide device according to Invention 4, in which the protection sheath includes a flange-shaped valve pressing part provided on an inner peripheral surface of the protection sheath, and the leakage prevention valve is interposed between the valve pressing part and a proximal end part of the inner sheath so that a position of the handle part in an axial direction is restricted.

### Invention 6

The medical instrument guide device according to any one of Inventions 1 to 5, in which an engaging part is provided to protrude on an outer peripheral surface of the inner sheath, the protection sheath includes a slit-shaped engaged part open toward a tube main body side, and the protection sheath is externally inserted into the inner sheath in a state in which rotation of the protection sheath is locked with respect to the inner sheath by engaging the engaged part and the engaging part with each other.

### Invention 7

The medical instrument guide device according to Invention 6, in which the port part is provided in the engaging part.

### Invention 8

The medical instrument guide device according to Invention 7, in which the tube main body includes a hardness change part that is arranged along a longitudinal direction of the tube main body and of which hardness is changed due to the fluid supplied and discharged from the port part, and the port part includes a fluid port for supplying and discharging the fluid to and from the tube main body, and a liquid port for supplying a liquid to an inner peripheral surface of the insertion passage.

### Invention 9

The medical instrument guide device according to Invention 8, in which the fluid port and the liquid port are installed adjacent to each other along an axial direction of the inner sheath.

### Invention 10

The medical instrument guide device according to Invention 9, in which the liquid port is arranged on a side opposite to a side of the inner sheath to which the tube main body is connected, with respect to the fluid port.

### Invention 11

The medical instrument guide device according to any one of Inventions 8 to 10, further comprising: a fluid tube connected to the fluid port; and a liquid tube connected to the liquid port, in which a pull-out direction of a proximal end side portion of the fluid tube from the fluid port and a pull-out direction of a proximal end side portion of the liquid tube from the liquid port are the same direction as each other.

### Invention 12

The medical instrument guide device according to Invention 11, in which the pull-out direction of the proximal end side portion of the fluid tube from the fluid port and the pull-out direction of the proximal end side portion of the liquid tube from the liquid port are directions perpendicular to the axial direction of the inner sheath.

### Invention 13

The medical instrument guide device according to any one of Inventions 1 to 12, in which the protection sheath includes a first sleeve part arranged on a tube main body side, and a second sleeve part arranged on a side opposite to the tube main body side with respect to the first sleeve part and having a larger outer diameter than the first sleeve part.

### Invention 14

The medical instrument guide device according to Invention 13, in which the first sleeve part includes a sleeve body large-diameter part arranged on the tube main body side, and a sleeve body small-diameter part arranged on a side opposite to the tube main body side with respect to the sleeve body large-diameter part and having a smaller outer diameter than the sleeve body large-diameter part.

### Invention 15

The medical instrument guide device according to Invention 14, in which an axial length of the sleeve body small-diameter part is longer than an axial length of the second sleeve part.

### Invention 16

The medical instrument guide device according to Invention 14 or 15, in which the sleeve body small-diameter part is arranged on a side opposite to the tube main body side with respect to the port part.

### Invention 17

The medical instrument guide device according to any one of Inventions 1 to 16, in which the tube main body includes a hardness change part that is arranged along a longitudinal direction of the tube main body and of which hardness is changed due to the fluid supplied and discharged from the port part, the port part includes a fluid port for supplying and discharging the fluid to and from the tube main body, a fluid tube connected to the fluid port is provided, a switching unit is provided in a middle of the fluid tube, and the switching unit includes a switching member capable of switching supply and discharge of the fluid for the fluid port.

### Invention 18

The medical instrument guide device according to Invention 17, in which the switching unit includes a fixing part attachably and detachably fixable to the medical instrument.

### Invention 19

The medical instrument guide device according to Invention 18, in which the medical instrument is an endoscope having an insertion part to be inserted into the body, and the fixing part includes a fitting part capable of being fitted into a treatment tool insertion part of the endoscope.

### Invention 20

The medical instrument guide device according to Invention 18 or 19, in which the switching member is arranged on a side opposite to a side of the fluid port with respect to the fixing part.

Although the example of the medical instrument guide device according to the embodiment of the present invention has been described above, the present invention may be improved or modified in several ways without departing from the scope of the present invention as defined by the claims.

### Explanation of References

10: guide tube
12: endoscope
14: insertion part
16: hand operating part
18: distal end hard part
18A: distal end surface
20: bendable part
22: soft part
24: illumination window
26: observation window
28: treatment tool outlet port
30: insertion port
32: treatment tool insertion part
33: bending prevention part
34: cap
35: cylindrical part
36: angle knob
40: vacuum pump
50: tube main body
50A: distal end part
50B: proximal end part
51: distal end opening part
52: outer tube
54: inner tube
56: inner space
58: shape deformable body
60: insertion passage
62: hydrophilic coating
64: adhesive
66: cap
68: adhesive
70: spiral tube
72: band-shaped member
74: high friction surface
76: adhesive
78: adhesive
80: sheet material
82: high friction surface
100: handle part
110: inner sheath
110A: distal end part
110B: proximal end part
112: port part
114: insertion passage
116: hydrophilic coating
118: connection portion
120: protection sheath
120A: distal end part
120B: proximal end part
122: proximal end side opening part
124: valve pressing part
126: first sleeve part
128: second sleeve part
130: step part
140: ring member
140A: proximal end surface
142: protrusion
144: pin
146: pin
148: pin
150: hole
152: hole
154: hole
160: leakage prevention valve
162: valve body part
164: tubular part
170: base part
172: protrusion part
180: slit
182: notch part
190: port
191: flow passage
192: tube
192A: tube
192B: tube
193: proximal end side portion
200: port
201: flow passage
202: tube
202A: distal end part
203: proximal end side portion
204: syringe
208: switching unit
210: three way stopcock
210A: first port
210B: second port
210C: third port
212: cock
214: hook
216: opening part
300: guide tube
302: protection sheath
304: first sleeve part
306: sleeve body large-diameter part
308: sleeve body small-diameter part
310: switching unit
312: hook
400: large intestine
402: sigmoid colon
404: transverse colon
406: lesion portion

## Claims

1. A medical instrument guide device having an insertion passage (114) for guiding a medical instrument (12) into a body, the medical instrument guide device comprising:
a long tube main body (50) to be inserted into the body; and
a handle part (100) provided on a proximal end side of the tube main body,
wherein the handle part includes an inner sheath (110) to which a proximal end part (110B) of the tube main body is connected and a protection sheath (120) externally inserted into the inner sheath, and
the inner sheath includes a port part (112) for supplying and discharging a fluid to and from the tube main body.

2. The medical instrument guide device according to claim 1,
wherein the tube main body includes a hardness change part that is arranged along a longitudinal direction of the tube main body and of which hardness is changed due to the fluid supplied and discharged from the port part.

3. The medical instrument guide device according to claim 1 or 2,
wherein a stress relaxation ring (140) that surrounds an outer periphery of a connection portion between the inner sheath and the tube main body in a loosely fitted state is arranged, and
the inner sheath and the protection sheath are coupled and fixed to the stress relaxation ring.

4. The medical instrument guide device according to any one of claims 1 to 3,
wherein a leakage prevention valve (160) that prevents a liquid from leaking from the tube main body is arranged inside the handle part.

5. The medical instrument guide device according to claim 4,
wherein the protection sheath includes a flange-shaped valve pressing part (124) provided on an inner peripheral surface of the protection sheath, and
the leakage prevention valve is interposed between the valve pressing part and a proximal end part of the inner sheath so that a position of the handle part in an axial direction is restricted.

6. The medical instrument guide device according to any one of claims 1 to 5,
wherein an engaging part (170) is provided to protrude on an outer peripheral surface of the inner sheath,
the protection sheath includes a slit-shaped engaged part open toward a tube main body side, and
the protection sheath is externally inserted into the inner sheath in a state in which rotation of the protection sheath is locked with respect to the inner sheath by engaging the engaged part and the engaging part with each other.

7. The medical instrument guide device according to claim 6,
wherein the port part is provided in the engaging part.

8. The medical instrument guide device according to claim 7,
wherein the tube main body includes a hardness change part that is arranged along a longitudinal direction of the tube main body and of which hardness is changed due to the fluid supplied and discharged from the port part, and
the port part includes
a fluid port for supplying and discharging the fluid to and from the tube main body, and
a liquid port for supplying a liquid to an inner peripheral surface of the insertion passage.

9. The medical instrument guide device according to claim 8,
wherein the fluid port and the liquid port are installed adjacent to each other along an axial direction of the inner sheath, and
preferably, the liquid port is arranged on a side opposite to a side of the inner sheath to which the tube main body is connected, with respect to the fluid port.

10. The medical instrument guide device according to claim 8 or 9, further comprising:
a fluid tube connected to the fluid port; and
a liquid tube connected to the liquid port,
wherein a pull-out direction of a proximal end side portion of the fluid tube from the fluid port and a pull-out direction of a proximal end side portion of the liquid tube from the liquid port are the same direction as each other, and
preferably, the pull-out direction of the proximal end side portion of the fluid tube from the fluid port and the pull-out direction of the proximal end side portion of the liquid tube from the liquid port are directions perpendicular to the axial direction of the inner sheath.

11. The medical instrument guide device according to any one of claims 1 to 10,
wherein the protection sheath includes
a first sleeve part (126) arranged on a tube main body side, and
a second sleeve part (128) arranged on a side opposite to the tube main body side with respect to the first sleeve part and having a larger outer diameter than the first sleeve part.

12. The medical instrument guide device according to claim 11,
wherein the first sleeve part includes
a sleeve body large-diameter part (306) arranged on the tube main body side, and
a sleeve body small-diameter part (308) arranged on a side opposite to the tube main body side with respect to the sleeve body large-diameter part and having a smaller outer diameter than the sleeve body large-diameter part.

13. The medical instrument guide device according to claim 12,
wherein an axial length of the sleeve body small-diameter part is longer than an axial length of the second sleeve part,
and/or
the sleeve body small-diameter part is arranged on a side opposite to the tube main body side with respect to the port part.

14. The medical instrument guide device according to any one of claims 1 to 13,
wherein the tube main body includes a hardness change part that is arranged along a longitudinal direction of the tube main body and of which hardness is changed due to the fluid supplied and discharged from the port part,
the port part includes a fluid port for supplying and discharging the fluid to and from the tube main body,
a fluid tube connected to the fluid port is provided,
a switching unit is provided in a middle of the fluid tube, and
the switching unit includes a switching member capable of switching supply and discharge of the fluid for the fluid port.

15. The medical instrument guide device according to claim 14,
wherein the switching unit includes a fixing part attachably and detachably fixable to the medical instrument, and
preferably,
the medical instrument is an endoscope having an insertion part to be inserted into the body, and the fixing part includes a fitting part capable of being fitted into a treatment tool insertion part of the endoscope,
and/or
the switching member is arranged on a side opposite to a side of the fluid port with respect to the fixing part.
